# EUROPEAN PATENT APPLICATION

(11) **EP 3 818 986 A1**
(43) Date of publication of application: **12.05.2021**
(21) Application number: 19831104.5
(22) Date of filing: 11.06.2019
(51) Int. Cl.: A61K 36/062, A23F 3/00, A23L 33/135, A61K 35/744, A61K 36/48, A61K 36/82, A61K 36/899, A61P 15/00, A61P 43/00

(54) **COMPOSITION FOR TREATMENT, PREVENTION, OR IMPROVEMENT OF MALE INFERTILITY**

(30) Priority: 02.07.2018 JP 2018126274
(71) Applicant: Mugen General Incorporated Association, Kirishima-shi, Kagoshima 899-6404 (JP)
(72) Inventor: YAMAMOTO, Masahiro, Kirishima-shi, Kagoshima 8996404 (JP)
(74) Representative: Mewburn Ellis LLP
(86) International application number: PCT/JP2019/023160
(87) International publication number: WO 2020/008821

(57) **Abstract**

Provided is a composition for use in medicines, food products, supplements, or beverages that can treat, prevent, or improve male infertility. The composition for treating, preventing, or improving male infertility of the present invention contains a koji mold fermentation product obtained by fermentation with a koji mold.

## Description

### Technical Field

The present invention relates to a composition for treating, preventing, or improving male infertility and foods using the composition.

### Background Art

Presently, it is reported that about 10% of the couples who desire pregnancy are infertility. Many females desire pregnancy at a higher age due to reasons including later marriage. As a consequence, it is pointed out that infertility cases tend to increase. Although a female-side cause, a male-side cause, or both of them are responsible for infertility, the male-side cause occupies 48% of the cases (Non Patent Literature 1). About 80 to 90% of male infertility are caused by spermatogenic dysfunction having problems in spermatogenesis in spermatogonia or sperm maturation. Specific symptoms thereof include oligospermia, azoospermia, teratospermia, and asthenospermia. Therefore, for treating such male infertility, sperm dysfunction has been studied.

Patent Literature 1 and Non Patent Literature 2 disclose that healthy sperms are rich in phospholipid hydroperoxide glutathione peroxidase (PHGPx, GPx4); and that if expression of GPx4 in sperms is reduced, a total number of sperms significantly decreases and motility of sperms decreases. Non Patent Literature 2 also discloses that a testis-specific GPx4 knockout mouse exhibits spermatogenic dysfunction equivalent to human male infertility.

Meanwhile, recently, traditional Japanese koji fermentation food products using koji, such as sake, shochu, amazake, miso, and soy sauce, have been re-recognized for value and popularity of koji is increasing. "Koji" means a product obtained by steaming rice, rice bran, wheat, bean or the like, and then allowing koji molds to proliferate therein. Among them, koji obtained by allowing koji mold to proliferate in rice is referred to as "rice koji (malted rice)". Koji molds are known to produce many types of enzymes, by the functions of which fermentation food products can be produced. Research is not limited to this field. The present inventor has conducted researches for finding new functions of koji molds and developing uses based on the functions. Patent Literature 2 reports that a feed obtained by fermenting, e.g., grains, with a koji mold, promotes growth of animals; whereas, Patent Literature 3 reports that a koji fermented composition, which is obtained by inoculating a koji mold into tea leaves and culturing the tea leaves, has an antioxidant activity.

### Citation List

### Patent Literature

Patent Literature 1: Japanese Patent Laid-Open No. 2004-344164
Patent Literature 2: Japanese Patent No. 6111416
Patent Literature 3: International Publication No. WO2015/119036

### Non Patent Literature

Non Patent Literature 1: "HUMAN + Men and Women Dictionary," [online], September 2014, edited by the Japan Society of Obstetrics and Gynecology, the internet <URL:http://www.jsog.or.jp/public/human_plus_dictionary>
Non Patent Literature 2: Hirotaka Imai, "Phospholipid hydroperoxide glutathione peroxidase (PHGPx) and male infertility," HORMONE FRONTIER IN GYNECOLOGY, 2012, Vol. 19, No. 2, p. 59-70

### Summary of Invention

### Technical Problem

As mentioned above, since a testis-specific GPx4 knockout mouse exhibits spermatogenic dysfunction equivalent to human male infertility, it has been expected to develop and provide materials for medicines and foods useful for treating, preventing, or improving male infertility by using the testis-specific GPx4 knockout mouse as a male infertility model mouse.

Note that, Patent Literature 2 and Patent Literature 3 separately disclose koji mold fermentation products obtained by fermenting grains and tea leaves with koji molds. However, whether or not the koji fermentation products and koji molds *per se* can be used for treating, preventing, or improving male infertility has not yet been studied, and thus, effectiveness of the products is not known.

Accordingly, the present invention was attained in view of the aforementioned circumstances. An object of the invention is to provide a composition for use in medicines, food products, supplements, or beverages that can treat, prevent, or improve male infertility.

### Solution to Problem

To solve the aforementioned problems, the composition for treating, preventing, or improving male infertility of the present invention comprises a koji mold fermentation product obtained by fermentation with a koji mold.

The koji mold fermentation product has functions of increasing a total number of sperms, decreasing the number of abnormal sperms having a structural defect (e.g., abnormal hairpin structure of sperm tail) or suppressing loss of spermatogenic cells in the testis tissue. Due to the function, the fermentation product is useful for treating, preventing, or improving male infertility. In the present invention, the "male" of the male infertility includes not only a human male but also a male of a non-human animal.

The koji mold fermentation product of the present invention is preferably obtained by using a material derived from tea leaves or rice as a fermentation feedstock. Based on this, a suitable feedstock for the koji mold fermentation product is selected.

The aforementioned koji mold is preferably black koji mold or white koji mold. Based on this, it is possible to select a suitable koji mold providing a koji mold fermentation product serving as an active ingredient of the present invention.

It is preferable that the composition for treating, preventing, or improving male infertility of the present invention further comprises a lactic acid bacterium. This promotes the proliferation of the koji mold and provides stable fermentation with the koji mold, to obtain a highly active koji mold fermentation product, and when the product will be taken, intestinal bacteria are properly regulated by the lactic acid bacterium, improving immunity.

In the present invention, the male infertility is preferably oligospermia or teratospermia. Based on this, a pathological condition to be suitably treated, prevented, or improved can be selected.

It is preferable that a food product for treating, preventing, or improving male infertility of the present invention comprises the aforementioned composition. Since a fermentation product by the koji mold, which is used for producing food products, is used as an active ingredient, the composition is highly safe and can be orally taken.

A method for producing a composition for treating, preventing, or improving male infertility of the present invention has the steps of inoculating a koji mold into a fermentation feedstock, and fermenting the fermentation feedstock with the koji mold to obtain a koji mold fermentation product; or has the steps of inoculating a koji mold and a lactic acid bacterium into a fermentation feedstock, and fermenting the fermentation feedstock with the koji mold and the lactic acid bacterium to obtain a koji mold fermentation product. In this manner, a composition for treating, preventing, or improving male infertility according to the present invention can be obtained. Since the koji mold and the lactic acid bacterium are co-cultured, proliferation of the koji mold is facilitated; fermentative treatment can be stably carried out; and the fermentation period is shortened. Note that, it is preferable that the fermentation feedstock is a material derived from tea leaves or rice. It is also preferable that the koji mold is black koji mold or white koji mold.

### Advantageous Effects of Invention

According to the present invention, it is possible to provide a composition for treating, preventing, or improving male infertility having the following excellent effects.
(1) The composition has an excellent treatment, prevention, or improvement effect on male infertility, increases the number of sperms in the semen, and simultaneously decreases the ratio of abnormal sperms.
(2) Since the composition comprises a fermentation product by a koji mold, which is used in producing food products, as an active ingredient, the composition is highly safe and easily taken.

### Brief Description of Drawings

[Figure 1] Figure 1 is a flowchart schematically showing a method for producing a koji mold fermentation product according to an embodiment of the present invention.
[Figure 2] Figure 2 is graphs showing mating results of control groups and test groups in Example 2.
[Figure 3] Figure 3 is a graph showing the total numbers of sperms of control groups and test groups in Example 3.
[Figure 4] Figure 4 is a graph showing the ratio of abnormal sperms of control groups and test groups in in Example 4.
[Figure 5] Figure 5 is a graph showing the ratio of the groups classified by sperm-tail fold angle in the sperms of control groups and test groups in Example 4.
[Figure 6] Figure 6 shows photographs of the testis tissues of control groups and test groups in Example 5.

### Description of Embodiments

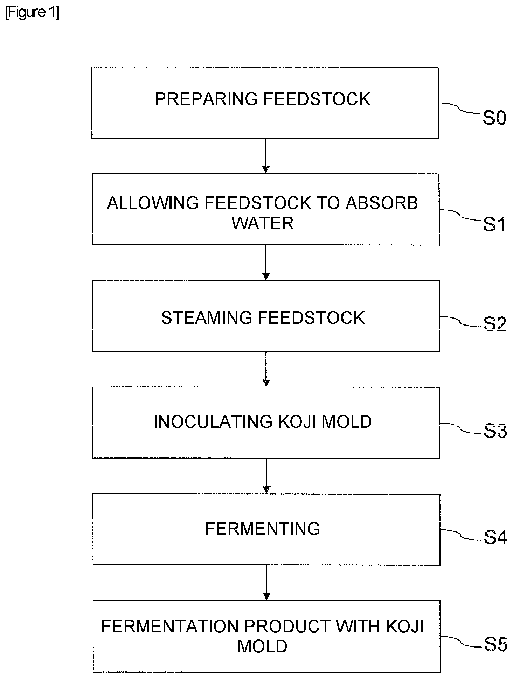

First, referring to Figure 1, the method for producing a koji mold fermentation product obtained by fermentation with a koji mold, comprised in the composition for treating, preventing, or improving male infertility of the present invention, will be described.

As shown in Figure 1, a method for producing a koji mold fermentation product according to an embodiment of the present invention is substantially constituted of step (S0) of preparing a fermentation feedstock, step (S1) of adding water to the fermentation feedstock to allow the feedstock to absorb water, step (S2) of steaming the fermentation feedstock, step (S3) of inoculating a koji mold into the steamed fermentation feedstock, step (S4) of fermenting the feedstock, and step (S5) of obtaining a koji mold fermentation product.

### (Preparation of fermentation feedstock)

First, the step (S0) of preparing a fermentation feedstock shown in Figure 1 will be described. In the present invention, as a fermentation feedstock, a plant-derived material is selected; for example, leaves, stems, roots, flowers, and fruits of plants, can be used. Of them, tea leaves, grains, or beans are suitably used. In particular, tea leaves or grains are suitably used as a fermentation feedstock, since they are excellent in male-infertility improvement effect. Examples of the grains include a wide variety of grains such as rice, corn, wheat, barley, rye, oats, sorghum, soba, and millets; however, rice is particularly preferable since its male-infertility improvement effect is excellent. As rice, not only polished white rice but also brown rice is included. As the tea leaves, not only leaves of a tea plant but also, e.g., stem and branch collected from a tea plant are included. As the tea leaves to be used as a fermentation feedstock for the koji mold fermentation product of the present invention, fresh tea leaves just collected can be suitably used as they are; however the tea leaves treated with heat immediately after collection, oolong tea or black tea obtained by oxidatively fermenting tea leaves collected with an oxidase contained therein, and post-fermented tea may be used. Furthermore, tea dregs remaining after infusion of the tea such as green tea, oolong tea, or black tea, can be used. Moreover, in order to enhance the efficiency of steaming treatment and culturing with a koji mold, it is preferable that tea leaves and tea stem are crushed into pieces of an appropriate size so as not to be excessively large.

### (Water absorption treatment)

The water absorption treatment step (S1) will be described. In this step, a fermentation feedstock is allowed to absorb water by adding water to the feedstock or soaking the feedstock in water. The amount of water to be absorbed, when, e.g., tea leaves are used as a fermentation feedstock, is controlled to fall within the range of 20 to 60% and preferably 30 to 50%, in terms of water content of the tea leaves.

### (Steaming treatment)

The steaming treatment step (S2) will be described. The fermentation feedstock, which was allowed to absorb water in the above step, is placed in a steamer. The steamer is covered with the lid and heated. In this manner, the fermentation feedstock can be heated by applying water vapor thereto. The time for the steaming treatment is preferably about 30 to 120 minutes, more preferably, 45 to 90 minutes, and particularly preferably about 60 minutes. By the steaming treatment, undesirable bacteria present in the feedstock are killed, with the result that fermentation in the later step can be carried out by a dominant species, a koji mold. Even if fresh tea leaves are used as a fermentation feedstock, if the steaming treatment is carried out, undesirable bacteria present in tea leaves are killed, and simultaneously, oxidase contained in the tea leaves are inactivated, with the result that the koji mold can be efficiently proliferated in tea leaves in the step later carried out. After completion of the steaming treatment, the feedstock is taken out from the steamer, uniformly spread on a table and cooled up to about 30 to 40°C. In this manner, the fermentation feedstock can be ready for inoculation with the koji mold.

### (Inoculation of koji mold)

The step (S3) of inoculating a koji mold into a fermentation feedstock will be described. In this step, the koji mold is inoculated into the fermentation feedstock cooled in the aforementioned steaming step. In the present invention, the koji mold refers to a microorganism for use in mainly producing koji fermentation food; more specifically, examples of the koji mold include black koji mold, white koji mold, and yellow koji mold. Of them, black koji mold refers to a group of molds belonging to the genus *Aspergillus* forming black or dark brown conidia (a kind of asexual spore) and used for producing distilled spirits such as Okinawa Awamori and Kagoshima potato shochu. Specific examples thereof include, but are not particularly limited to, *Aspergillus awamori var. kawachii* (kawachii black koji mold), *Aspergillus luchuensis, Aspergillus awamori, Aspergillus saitoi, Aspergillus inuii, Aspergillus usamii* and *Aspergillus aureus.* White koji mold refers to a group of molds belonging to the genus *Aspergillus* forming white-yellow conidia. Specific examples thereof include *Aspergillus kawachii.* Yellow koji mold refers to a group of molds belonging to the genus *Aspergillus* forming yellow or yellow-green conidia and is a microorganism mainly used for producing, e.g., sake, miso, and soy sauce. Specific examples thereof include, but are not particularly limited to, *Aspergillus oryzae* and *Aspergillus sojae.* In the present invention, black koji mold or white koji mold is preferably used since it is excellent in male-infertility improvement effect. Of them, black koji mold, i.e., *Aspergillus awamori var. kawachii, Aspergillus luchuensis,* or *Aspergillus inuii,* white koji mold, i.e., *Aspergillus kawachii,* and combination of these can be suitably used, particularly, *Aspergillus awamori var. kawachii* is preferable. In inoculation, the koji mold is uniformly applied onto a fermentation feedstock spread on a table, followed by mixing. In inoculation of the koji mold, the spores are preferably inoculated, and preferably added and inoculated in a ratio of 500,000 spores or more and preferably 2,000,000 spores per gram (dry weight) of a fermentation feedstock. For example, if the number of spores contained in seed malt (per g) is 2 billion, about 1.0 g (0.1 wt%) of the seed malt, may be added to a fermentation feedstock (per kg). After the koji mold is inoculated into a fermentation feedstock, the feedstock is preferably mixed well to disperse the koji mold all over the feedstock.

In the inoculation step (S3) of a koji mold, it is preferable that a lactic acid bacterium is further inoculated to co-culture the koji mold and the lactic acid bacterium. If the koji mold and the lactic acid bacterium are co-cultured, proliferation of the koji mold is facilitated and the fermentative treatment can be stably carried out. In addition, since these inoculated species are dominated, proliferation of undesirable bacteria is suppressed. The lactic acid bacterium which can be used herein include lactic acid bacteria used in lactic acid bacterium fermentation foods. Examples thereof include bacteria of the genus *Lactobacillus* such as *Lactobacillus casei, Lactobacillus fermentum* or *Lactobacillus bulgaricus; bifidobacteria* such as *Bifidobacterium bifidum;* bacteria of the genus *Lactococcus* such as *Lactococcus lactis;* and bacteria of the genus *Enterococcus* such as *Enterococcus faecium.* The lactic acid bacterium to be inoculated is previously prepared by culturing and added in an amount of about 0.1 wt% in terms of bacterial cells per fermentation feedstock (kg). A koji mold fermentation product obtained by fermentation with a koji mold and a lactic acid bacterium contains not only various enzymes produced by the koji mold and the lactic acid bacterium but also the koji mold cells and its spores and the lactic acid bacterium cells. Since the lactic acid bacterium has a probiotic function, if it is taken, the intestinal environment can be regulated; immune function is augmented; and a male-infertility improvement effect is expected.

### (Fermentation)

The fermentation step (S4) carried out by culturing the koji mold inoculated into the fermentation feedstock will be described. In this step, the koji mold in the feedstock inoculated therewith is proliferated. First, the fermentation feedstock to which the koji mold is added is placed in a culture chamber kept at about 30°C. Fermentation proceeds with passage of time and the temperature of the fermentation feedstock (e.g., tea leaves) increases. If the temperature thereof exceeds 40°C, it is usually difficult for the koji mold to proliferate. Thus, air is supplied to reduce the temperature of the feedstock. The volume of air is controlled so as to maintain the temperature of the feedstock at 30°C to 42°C and preferably 30°C to 40°C. Note that, the fermentation temperature is appropriately controlled depending on the type of the koji mold to be used for fermentation. More specifically, if black koji mold is selected as the koji mold, for example, air is supplied from initiation of culture up to 12 to 30 hours so as to control the temperature of a fermentation feedstock (e.g., tea leaves) to fall within the range of 35°C to 40°C, and thereafter, the volume of air is varied to fall the temperature within a slightly lower temperature range of 30°C to 35°C. At the total culture period of 1 to 4 days, preferably 36 to 72 hours and further preferably 40 to 60 hours from initiation of culture, culture is completed to obtain a koji mold fermentation product.

Note that, in steps S1 to S4 of producing a koji mold fermentation product, washing of a feedstock, water absorption, steaming, inoculation and fermentation (koji making) of the koji mold can be carried out in the same apparatus (for example, drum-system automatic koji making device).

### (Koji mold fermentation product)

The resultant koji mold fermentation product is so-called "koji," that is a proliferated koji mold in a fermentation feedstock. The koji mold fermentation product obtained in the aforementioned steps contains a certain content of moisture. Moisture can be removed by air-drying or low temperature dehumidification/drying. The koji mold fermentation product dewatered can be crushed into powders and granules. The koji mold fermentation product, even if it is once dewatered, effectively maintains its function. In other words, the koji mold fermentation product has a treatment, prevention, or improvement effect on male infertility. When tea leaves are used as a fermentation feedstock, since the tea leaves originally have a cell growth inhibitory activity, they are unsuitable for oral intake; however, the koji mold fermentation product of the present invention, since it is reduced in cell growth inhibitory activity, can be orally taken.

The composition for treating, preventing, or improving male infertility of the present invention comprises the koji mold fermentation product obtained by fermentation with a koji mold as mentioned above, as an active ingredient. Thus, the composition improves, prevents, or treats male infertility based on spermatogenic dysfunction such as oligospermia, azoospermia, teratospermia, and asthenospermia and has a suppressive effect on the onset of spermatogenic dysfunction. Thus, the composition comprising the koji mold fermentation product of the present invention as an active ingredient can be used as medicines, quasi drugs and food compositions for preventing, treating, or improving these diseases. Although male infertility is caused by unknown causes, the composition according to the present invention treats, prevents, or improves male infertility cases developed by various causes. In particular, the composition can suitably treat, prevent, or improve male infertility caused by reduction in expression of GPx4 in sperms. A target for the composition for treating, preventing, or improving male infertility of the present invention is mainly a human male; however, the composition can be used for males of wide variety of non-human animals in the form of veterinary medicines, animal supplements, animal feeds, or pet foods.

The dosage of the composition for treating, preventing, or improving male infertility of the present invention varies depending on the desired prevention or therapeutic effect, administration method, age, body weight, or the like. Because of this, the dosage is not simply defined; however, the oral dosage of the koji mold fermentation product per day is usually about 0.1 to 1000 mg/kg body weight, preferably about 0.5 to 200 mg/kg body weight, and further preferably about 1 to 50 mg/kg body weight. The dosage may be divided into 1 to 3 portions and administered.

The composition for treating, preventing, or improving male infertility of the present invention can be prepared in various dosage forms by methods conventionally used. In this case, dosage forms can be prepared by using additives such as a carrier and an excipient for general pharmaceutical preparation, which are pharmaceutically acceptable additives. In order to improve bioavailability and stability of the koji mold fermentation product according to the present invention, a drug delivery system including techniques for preparing drug products such as encapsulation using micro capsules, micronization, and inclusion using, e.g., cyclodextrin, can be used.

The above composition can be used in a dosage form such as tablets, granules, capsules, or oral liquids; however, the composition is preferably used in a dosage form suitable for absorption through the digestive tract. When the preparation is provided in a desirable dosage form in view of, e.g., distribution and storability, a conventional technique for preparing a drug product can be used.

The composition for treating, preventing, or improving male infertility of the present invention can be used as a food composition in the form of a supplement such as tablets, capsules, granules and syrups; and various forms used in beverages, confectioneries, breads, rice gruels, serials, noodles, jellies, soups, dairy products, and seasonings. When the composition is used as a food composition, other active ingredients and nutrients such as vitamins, minerals or amino acids can be added in various combinations as long as they do not influence on the efficacy of the active ingredient of the present invention. Examples of food products developed from the food composition of the present invention include supplements, healthy foods, functional foods, and foods for specified health use. The ingestion dose of the food composition of the present invention per day in terms of the koji mold fermentation product is preferably about 0.1 to 1000 mg/kg body weight, more preferably about 0.5 to 200 mg/kg body weight, and further preferably about 1 to 50 mg/kg body weight. The ingestion dose is preferably divided into 1 to 3 portions and taken.

Now, the present invention will be more specifically described by way of Examples; however, the present invention is not limited by Examples.

### Examples

### [Example 1]

### 1. Preparation of koji mold fermentation product

In this Example, fresh tea leaves were used as a fermentation feedstock. After a water absorption treatment was applied to tea leaves by adding water such that the moisture content of tea leaves becomes about 50%, the tea leaves were put in a drum-system koji making device, and then, a steaming treatment was carried out in the conditions of 95°C and 60 minutes. After the tea leaves were cooled up to 30°C in the drum-system koji making device, seed malt (number of spores 2 billion/seed malt (gram)) of kawachii black koji mold (*Aspergillus awamori var. kawachii*) and *Lactobacillus casei* were added to the tea leaves such that the concentrations of both of them became 0.1 wt% and mixed to make koji in the drum-system koji making device over 3 days. The temperature during making koji was controlled as follows. After the seed malt was added, air was supplied for 24 hours to control the temperature of the tea leaves to fall in the range of 35°C to 40°C. For the following 48 hours, the amount of air to be supplied was controlled such that the temperature of the tea leaves falls in a slightly lower temperature range of 30°C to 35°C. A koji mold fermentation product produced over 3 days in the device was taken out and crushed to obtain a powdery koji mold fermentation product.

### [Example 2]

### 2. Improvement of male infertility by koji mold fermentation product

In this Example, as a male infertility model mouse, a male testis-specific non-mitochondrial GPx4 knockout mouse was used (see, Non Patent Literature 2). The testis-specific non-mitochondrial GPx4 knockout mouse is a mouse deficient in non-mitochondrial phospholipid hydroperoxide glutathione peroxidase (cGPx4) in the testis and has the phenotypes listed in the following Table 1.

**[Table 1]**

| Number of sperms | Sperm motility | Sperm shape | Testis tissue | Reproduction ability |
|---|---|---|---|---|
| Decrease | Decrease * | Abnormal hairpin structure (fold) in sperm tail * | Partial removal of spermatocytes | Tendency of infertility, 50% fertility of normal |

| | | | | |
|---|---|---|---|---|
| *: Normal sperms are present in a ratio of about 20% | | | | |

A single 3 weeks-old male testis-specific non-mitochondrial GPx4 knockout mouse was assigned to each of the four groups shown in Table 2 (below) and separately raised (singly per cage) up to 8 weeks-old. To a normal control group, mouse breeding feed CE-2 (CLEA Rodent Diet CE-2; a product manufactured by CLEA Japan, Inc.) was fed during the test period. As a positive control substance, vitamin E (Non Patent Literature 1), which is used in treatment for male infertility, was selected and a mixture of feed CE-2 and vitamin E was fed to a positive control substance administration group during the test period. The ratio of vitamin E in the mixture was 100 mg relative to 100 g of feed CE-2. To the test groups, a mixture of feed CE-2 and koji mold fermentation product obtained in Example 1 was fed during the test period. For a low dose group, a feed having a content of koji mold fermentation product of 0.5 wt% was used. For a high dose group, a feed having a content of koji mold fermentation product of 5.0 wt% was used. Feed CE-2 and feed CE-2 mixed with the positive control substance or a koji mold fermentation product were free to take and water was free to drink from a water-supply bottle. In the aforementioned conditions, mice were raised from 3 weeks-old to 8 weeks-old.

**[Table 2]**

| Control group and test group (single male × 3 females) | | Detailed administration |
|---|---|---|
| Control group | Normal control group "CE-2" | Non administration (CE-2 feed, alone) |
| | Positive control substance administration group "VE high-content feed" | Vitamin E (high-addition)(vitamin E 100 mg + CE-2 feed 100 g) |
| Test group | koji mold fermentation product administration group (low dose) "tea koji 0.5%" | koji mold fermentation product 0.5 wt% (koji mold fermentation product 0.5 g + CE-2 feed 99.5 g) |
| | koji mold fermentation product administration group (high dose) "tea koji 5.0%" | koji mold fermentation product 5.0 wt% (koji mold fermentation product 5 g + CE-2 feed 95 g) |

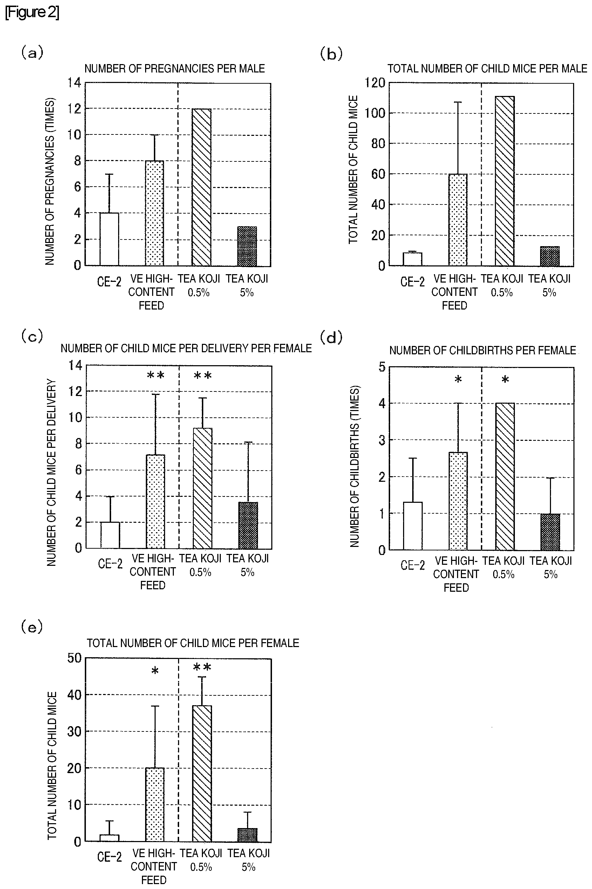

When male testis-specific non-mitochondrial GPx4 knockout mice grew up to be crossable (8 weeks-old) mice, three of 7 weeks-old BALB/c female mice were assigned to each cage and crossed with a single male testis-specific non-mitochondrial GPx4 knockout mouse. The mating period was 3 months. After initiation of mating, the feed that was given to the male mouse from 3 weeks-old per cage was continuously fed to control groups or test groups. To the female mice assigned per cage, the same feed as that given to the male mouse was fed. The number of pregnancies and the number of child mice per female mouse during the mating period were examined. The results are shown in Figure 2. Reference symbol * above the bar of graphs in Figure 2 represents that "p value" when compared to a normal control group "CE-2," satisfies p < 0.05; and ** represents that "p value" satisfies p < 0.01.

In normal control group "CE-2", the total number of pregnancies of three female mice was 4 (Figure 2a) and the total number of child mice thereof was less than 10 (Figure 2b). The number of child mice per delivery of the female mice was as small as 2 (Figure 2c). In the results of the positive control substance (vitamin E) administration group "VE high-content feed," the total number of pregnancies of three female mice was 8 (Figure 2a); the total number of child mice thereof was 60 (Figure 2b); and the number of child mice per delivery of the female mice was as large as 7 (Figure 2c). An improvement in infertility tendency of male testis-specific non-mitochondrial GPx4 knockout mouse was observed. In contrast, in koji mold fermentation product low-dose group "tea koji 0.5%," it was confirmed that the total number of pregnancies of three female mice was 12 (Figure 2a); and that the total number of child mice thereof exceeds 110 (Figure 2b). In consideration that the pregnancy period of BALB/c female mouse is about 3 weeks; and that the mating period is 3 months, the number of pregnancies per female mouse is at most 4. In this experiment, all the three female mice assigned to the cage of each test group were found to be pregnant 4 times. In addition, the number of child mice per delivery of the female mice was as large as about 9 (Figure 2c). From the results, it was demonstrated that the koji mold fermentation product of the present invention greatly improves infertility tendency of a male infertility model mouse and can treat male infertility.

Note that, in koji mold fermentation product high-dose group "tea koji 5%," the total number of pregnancies of three female mice decreased to 3 (Figure 2a); the total number of child mice thereof was as small as about 10 (Figure 2b); and the number of child mice per delivery of the female mice was about 4 (Figure 2c). From the results, it was found that the dosage of koji mold fermentation product is preferably lower than that of "tea koji 5%" of the high dose group. Note that, provided that the body weight of a male mouse is about 25 g, daily intake of feed is 3 to 4 g/day, the dosage of a koji mold fermentation product in the high dose group is 6 to 8 g/kg body weight/day; whereas, the dosage of a koji mold fermentation product in the low dose group is 0.6 to 0.8 g/kg body weight/day. Thus, in order to improve male infertility, it was presumed that the dosage of a koji mold fermentation product is preferably less than 6 g/kg body weight/day, more preferably, 3 g/kg body weight/day or less, and further preferably 1 g/kg body weight/day or less.

### [Example 3]

### 3. Improvement of the number of sperms by koji mold fermentation product

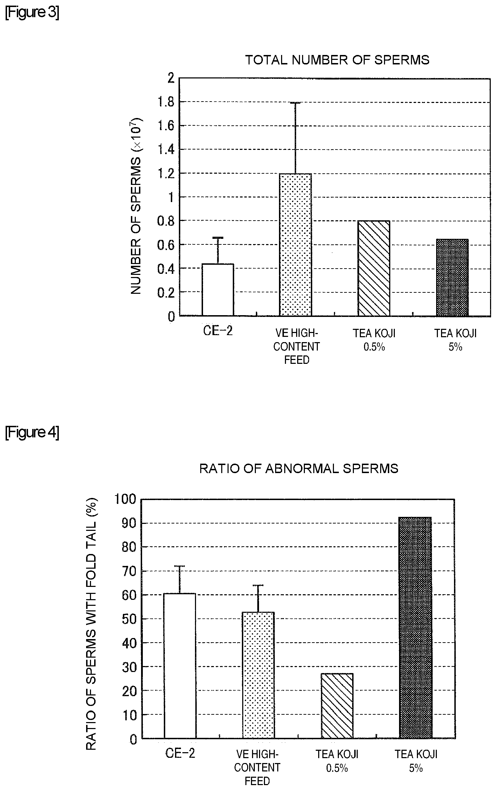

The number of sperms in the testis-specific non-mitochondrial GPx4 knockout mouse is reduced to a half or less of that of a normal mouse. Then, the effect of intake of the koji mold fermentation product of the present invention on the number of sperms was examined, as follows. After the mating period in Example 1 was terminated, each of the male testis-specific non-mitochondrial GPx4 knockout mice of the control groups and test groups was dissected and the epididymis was taken out. Sperms were collected from each epididymis, as follows. In a 24-well petri dish in which TYH culture medium (500 µL) was placed in advance, the epididymis was placed. A cut was made in the epididymis so as for sperms to move to the culture medium and the sperms were cultured at 37°C for 2 hours. The resultant sperm suspension was collected in tubes and centrifuged at 4°C and 2000 rpm for 5 minutes. After the supernatant was removed, PBS (500 µL) was added to the tubes, which were centrifuged at 4°C and 2000 rpm for 5 minutes, and then, the supernatant was removed. This operation was repeated twice to wash the sperms. Thereafter, PBS (500 µL) was added to the sperms to obtain a test sperm fluid. The test sperm fluid was diluted 50 fold with PBS and the number of sperms was counted by a hemocytometer. The results are shown in Figure 3.

According to the graph of Figure 3, in testis-specific non-mitochondrial GPx4 knockout mice of the koji mold fermentation product low-dose group, "tea koji 0.5%," it was found that the number of sperms is about 2 times as high as that of the mice of the normal control group "CE-2." Note that, in the mice of the koji mold fermentation product high-dose group, "tea koji 5%," the number of sperms was high; however, it was found that the number of sperms of the low dose group "tea koji 0.5%" is larger than that of "tea koji 5%". From the results, it was demonstrated that the koji mold fermentation product of the present invention can improve oligospermia causing male infertility.

### [Example 4]

### 4. Improvement of abnormal sperms by koji mold fermentation product

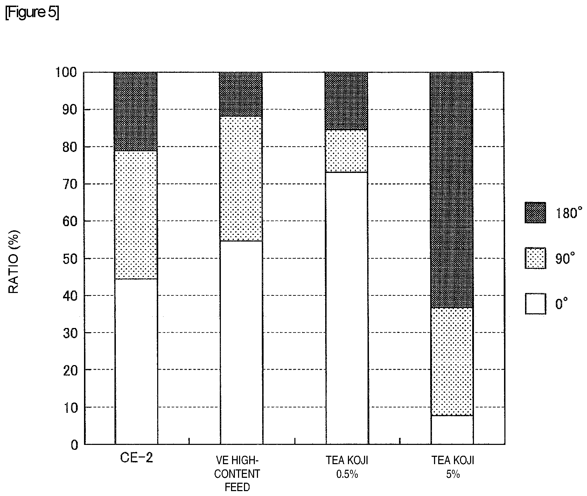

In a testis-specific non-mitochondrial GPx4 knockout mouse, a half or more of the sperms have an abnormal structure (a sperm tail is folded to form a hair-pin structure); in other words, abnormal sperm morphology. Then, the effect of intake of the koji mold fermentation product of the present invention on sperm morphology was examined, as follows. The test sperm fluid collected in Example 3 was diluted 10 fold with PBS. Ten µL of the dilution was taken and dropped on a slide glass, and then, a cover glass was placed thereon. The shapes of the sperms were observed by an all-in-one microscope (KEYENCE CORPORATION). The sperms in a plurality of fields of view under the microscope were photographed, and the numbers of normal sperms and abnormal sperms in the photographs were counted, and then, an average ratio of abnormal sperms was calculated. Note that, the abnormal sperms was classified into groups by sperm-tail fold angle as follows: A group of the sperms whose tails did not fold (straight shape) expressed as "0°"; a group of the sperms whose tails folded or folded at an angle of about 90° expressed as "90°" and a group of the sperms whose tails folded about 180° expressed as "180°." In this manner, the numbers of sperms of the groups were counted. Figure 4 shows the abnormal sperm ratio of the control groups and test groups; and Figure 5 shows the ratio of the groups classified by sperm-tail fold angle.

According to the graph shown in Figure 4, it was found that in the testis-specific non-mitochondrial GPx4 knockout mice of the koji mold fermentation product low-dose group "tea koji 0.5%," the abnormal sperm ratio was extremely low compared to the mice of normal control group "CE-2." The significantly low ratio of the abnormal sperms was an advantageous effect that was not observed in mice of the positive control substance (vitamin E) administration group, "VE high-content feed." The results demonstrated that the koji mold fermentation product of the present invention improves teratospermia causing male infertility. Note that, it was confirmed that the abnormal sperm ratio of the mice of the koji mold fermentation product high-dose group "tea koji 5%" was high compared to that of the mice of the normal control group "CE-2." From the results, it was found that the dose of koji mold fermentation product is preferably lower than the dose of the high dose group "tea koji 5%."

According to the graph shown in Figure 5, in the testis-specific non-mitochondrial GPx4 knockout mice of the koji mold fermentation product low-dose group "tea koji 0.5%," the ratio of the group having an abnormal sperm tail structure classified as sperm-tail fold angle of about "90°" is low. From this, it was confirmed that structural defect of sperm tails is improved by the koji mold fermentation product.

### [Example 5]

### 5. Improvement of testis tissue by koji mold fermentation product

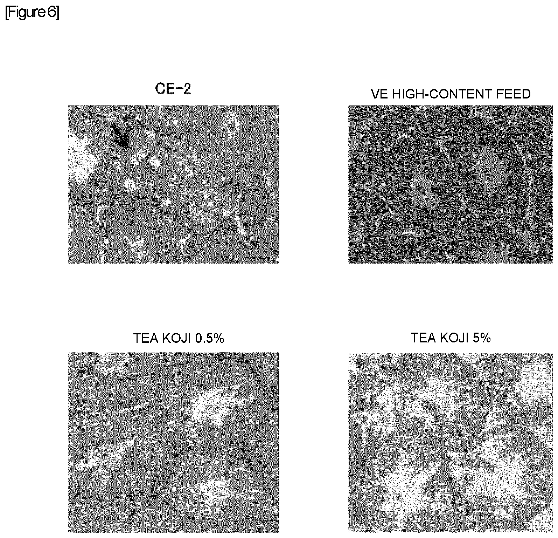

In the testis-specific non-mitochondrial GPx4 knockout mice, removal of spermatocytes from the testis tissue is observed. Then, the effect of intake of the koji mold fermentation product of the present invention on the spermatocytes was examined, as follows. After termination of the mating period in Example 1, each of the male testis-specific non-mitochondrial GPx4 knockout mice in the control groups and test groups was dissected and the testis was excised out. The tissue of the testis was fixed with a 10 % neutral formalin solution, embedded in paraffin, sectioned into 4-µm slices in thickness, stained with hematoxylin eosin, and subjected to observation. The photographs of the spermatocytes in the testis tissues of the control groups and test groups are shown in Figure 6.

In Figure 6, in the photograph of the normal control group "CE-2," the arrow points out the site from which spermatocytes are removed. In the koji mold fermentation product low-dose group "tea koji 0.5%" similarly to the positive control substance administration group "VE high-content feed," removal of the spermatocytes was improved and the cells had a regular shape. In the koji mold fermentation product high-dose group "tea koji 5%," a partial removal of spermatocytes was found. From the results, it was confirmed that the dose of the koji mold fermentation product is preferably lower than that of the high dose group "tea koji 5%."

The present invention is not limited by the embodiments or Examples mentioned above. Various design modifications are included in the technical scope as long as they can be made without departing from the scope of the invention claimed in the claims of the invention.

### Industrial Applicability

The present invention provides a composition for treating, preventing, or improving male infertility. Thus, the invention is useful in a wide variety of industries including the fields of medicine, food products (including supplement, healthy food, functional food, food for specified health use).

## Claims

1. A composition for treating, preventing, or improving male infertility, comprising a koji mold fermentation product obtained by fermentation with a koji mold.

2. The composition for treating, preventing, or improving male infertility according to claim 1, wherein the koji mold fermentation product is obtained by using a material derived from tea leaves or rice as a fermentation feedstock.

3. The composition for treating, preventing, or improving male infertility according to claim 1 or 2, wherein the koji mold is black koji mold or white koji mold.

4. The composition for treating, preventing, or improving male infertility according to any one of claims 1 to 3, further comprising a lactic acid bacterium.

5. The composition for treating, preventing, or improving male infertility according to any one of claims 1 to 4, wherein the male infertility is oligospermia or teratospermia.

6. A food product for treating, preventing, or improving male infertility, comprising the composition according to any one of claims 1 to 4.

7. A method for producing a composition for treating, preventing, or improving male infertility, comprising the steps of inoculating a koji mold into a fermentation feedstock, and fermenting the fermentation feedstock with the koji mold to obtain a koji mold fermentation product.

8. A method for producing a composition for treating, preventing, or improving male infertility, comprising the steps of inoculating a koji mold and a lactic acid bacterium into a fermentation feedstock, and fermenting the fermentation feedstock with the koji mold and the lactic acid bacterium to obtain a koji mold fermentation product.

9. The method for producing a composition for treating, preventing, or improving male infertility according to claim 7 or 8, wherein the fermentation feedstock is a material derived from tea leaves or rice.

10. The method for producing a composition for treating, preventing, or improving male infertility according to any one of claims 7 to 9, wherein the koji mold is black koji mold or white koji mold.
